# EUROPEAN PATENT SPECIFICATION

(11) **EP 1 893 252 B1**
(45) Date of publication and mention of the grant of the patent: **08.06.2011**
(21) Application number: 06750175.9
(22) Date of filing: 13.04.2006
(51) Int. Cl.: A61M 1/00, A61M 31/00, A61M 5/00

(54) **REFLUX CONTROL IN MICROSURGICAL SYSTEM**
REFLUX-STEUERUNG IN EINEM MIKROCHIRURGISCHEN SYSTEM
CONTROLE DU REFLUX DANS UN SYSTEME MICROCHIRURGICAL

(30) Priority: 21.06.2005 US 157714
(43) Date of publication of application: 05.03.2008
(73) Proprietor: Alcon, Inc., 6331 Hünenberg (CH)
(72) Inventor: TODD, Kirk, W., Yorba Linda, California 92887 (US); HOPKINS, Mark, A., Mission Viejo, California 92691 (US)
(74) Representative: Moore, Barry
(86) International application number: PCT/US2006/014066
(87) International publication number: WO 2007/001592

(56) References cited:
- US-A- 4 865 584
- US-A- 5 647 853
- US-A- 5 697 898
- US-A1- 2001 023 345

## Description

### Field of the Invention

The present invention generally pertains to controlling reflux in microsurgical systems and more particularly to controlling reflux in ophthalmic microsurgical systems.

### Description of the Related Art

During small incision surgery, and particularly during ophthalmic surgery, small probes are inserted into the operative site to cut, remove, or otherwise manipulate tissue. During these surgical procedures, fluid is typically infused into the eye, and the infusion fluid and tissue are aspirated from the surgical site. These probes have small orifices that are easily clogged with tissue. Such clogging is typically referred to as "occlusion", "tip occlusion", or "port occlusion". The process of clearing such occlusions is typically referred to as "reflux".

A traditional method of reflux is to create a backpressure pulse of fluid that travels through the aspiration circuit to the tip or port of the probe to clear the incarcerated tissue. The US Patent US 569 7898, which is directed to an apparatus for removing a cataract from the eye, teaches such a method. Because a single pulse of fluid often does not clear the occlusion, a surgeon must typically utilize a series of pulses until he or she visually observes the tip or port of the probe to be clear through the operating microscope. Despite this method of clearing occlusion, a need continues to exist for an improved method of controlling reflux in a microsurgical system.

### Summary of the Invention

The present invention is a method of controlling reflux in a microsurgical system. A pump is operated to provide irrigating fluid at a constant flow rate to an occluded port of a surgical device. A reflux pressure is sensed proximate the port over time. The reflux pressure is monitored as a function of time to detect an occlusion break.

### Brief Description of the Drawings

For a more complete understanding of the present invention, and for further objects and advantages thereof, reference is made to the following description taken in conjunction with the accompanying drawings, in which:
Figure 1 is a schematic diagram illustrating certain portions of an infusion circuit of a microsurgical system; and
Figure 2 schematically illustrates reflux pressure at a constant flow rate in the infusion circuit of Figure 1.

### Detailed Description of the Preferred Embodiments

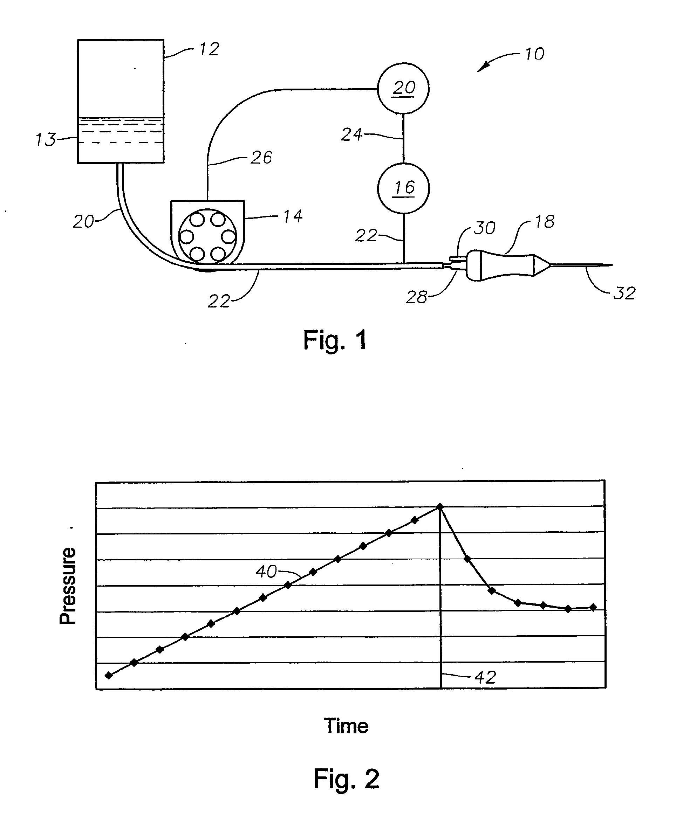

The preferred embodiment of the present invention and its advantages is best understood by referring to Figures 1-2 of the drawings. As shown in Figure 1, microsurgical system 10 includes an infusion fluid reservoir 12, a pump 14, a pressure transducer 16, a surgical device 18, and a computer or microprocessor 20. Reservoir 12 contains a surgical irrigating fluid 13, such as BSS PLUS® intraocular irrigating solution available from Alcon Laboratories, Inc. A fluid line 20 fluidly couples reservoir 12 and pump 14, and a fluid line 22 fluidly couples pump 14, pressure transducer 16, and surgical device 18. An interface 24 electrically couples pressure transducer 16 and microprocessor 20, and an interface 26 electrically couples microprocessor 20 and pump 14.

Pump 14 may be any suitable device for generating pressure or vacuum but is preferably a peristaltic pump, a scroll pump, or a vane pump. Pressure transducer 24 may be any suitable device for directly or indirectly measuring pressure or vacuum. Surgical device 18 may be any microsurgical instrument, probe, or handpiece but is preferably an ultrasound probe, a phacoemulsification probe, a liquefracture handpiece, an aspiration probe, or a vitrectomy probe. As shown in Figure 1, surgical device 18 is a vitrectomy probe. Surgical device 18 has an infusion port 28 for fluidly coupling with fluid line 22, an aspiration port 30 for fluidly coupling with an aspiration circuit (not shown) of microsurgical system 10, and a tip or port 32. Microprocessor 20 is capable of implementing feedback control, and preferably PID control.

In operation, aspirated fluid and tissue are provided to the aspiration circuit of microsurgical system 10 via aspiration port 30. An occlusion of port 30 may be detected by a surgeon through the operating microscope or automatically by microprocessor 20. Upon occlusion detection, microprocessor 20 sends an appropriate signal to pump 14 via interface 26 to rotate at a constant speed to provide irrigating fluid 13 at a constant flow rate to port 32. Pressure sensor 16 repeatedly senses the reflux pressure in fluid line 22 (and thus port 32) in real time and provides a corresponding signal to microprocessor 20 via interface 24. As shown in Figure 2, as long as port 32 remains occluded, the reflux pressure vs. time curve or function 40 created by pressure sensor 16 and microprocessor 20 will have a positive slope. At occlusion break 42 (when port 32 is cleared), curve 40 will exhibit either a negative or zero slope. Microprocessor 20 monitors curve 40 to detect occlusion break 42. Upon occurrence of occlusion break 42, microprocessor 20 sends a signal to pump 14 to cease providing reflux pressure.

From the above, it may be appreciated that the present invention provides an improved method of controlling reflux in a microsurgical system. In contrast to traditional reflux, only a single reflux cycle is required to clear port 32, and only the exact pressure and volume of irrigating fluid 13 required to clear port 32 is provided by pump 14. Therefore, occlusion is cleared more quickly, and reflux is safer for the patient.

The present invention is illustrated herein by example, and various modifications may be made by a person of ordinary skill in the art. For example, while the present invention is described above relative to controlling reflux in an ophthalmic microsurgical system, it is also applicable to other microsurgical systems.

It is believed that the operation and construction of the present invention will be apparent from the foregoing description. While the apparatus and methods shown or described above have been characterized as being preferred, various changes and modifications may be made therein without departing from the scope of the invention as defined in the following claims.

## Claims

1. A computer implemented method of controlling reflux in a microsurgical system (10), **characterized in that** it comprises the steps of:
Providing a signal to effect operation of a pump (14) to provide irrigating fluid (13) at a constant flow rate to an occluded port (32) of a surgical device (18);
Using a pressure sensor (16) to sense a reflux pressure proximate said port (32) over time; and
Monitoring said reflux pressure as a function of time to detect an occlusion break.

2. The method of claim 1 wherein said monitoring step comprises monitoring a slope of a reflux pressure versus time curve (40).

3. The method of claim 2 wherein said step of monitoring said slope comprises detecting a change in a sign of said slope.

4. The method of claim 3 wherein said slope changes from a positive sign to a negative sign.

5. The method of claim 3 wherein said slope changes from a positive sign to zero.

6. The method of claim 1 wherein said operating step commences upon detection of an occlusion of said port.

7. A computer program which when run on a microprocessor (20) is configured to carry out the method steps of any one of claims 1 to 6.

## Patentansprüche

1. Computerimplementiertes Verfahren zum Steuern des Rückflusses (Reflux) in einem mikrochirurgischen System (10),
**dadurch gekennzeichnet, dass** es aufweist:
Bereitstellen eines Signals, um den Betrieb einer Pumpe (14) zu bewirken, die Spülflüssigkeit mit einem konstanten Durchfluss an einen verstopften Anschluss (32) eines chirurgischen Geräts (18) liefert;
Verwenden eines Drucksensors (16), um einen Reflux-Druck in der Nähe des Anschlusses (32) über der Zeit zu erfassen; und
Überwachen des Reflux-Drucks in Abhängigkeit von der Zeit, um das Aufbrechen der Verstopfung zu erkennen.

2. Verfahren nach Anspruch 1, bei dem der Überwachungsschritt das Überwachen einer Steigung einer Kurve (40) des Reflux-Drucks über der Zeit aufweist.

3. Verfahren nach Anspruch 2, bei dem der Überwachungsschritt das Erkennen der Änderung des Vorzeichens einer Steigung aufweist.

4. Verfahren nach Anspruch 3, bei dem sich eine Steigung von einem positiven zu einem negativen Vorzeichen ändert.

5. Verfahren nach Anspruch 3, bei dem sich eine Steigung von einem positiven Vorzeichen zu null ändert.

6. Verfahren nach Anspruch 1, bei dem der Schritt der Inbetriebnahme bei der Detektion einer Verstopfung des Anschlusses beginnt.

7. Computerprogramm, das so konfiguriert ist, dass es bei Ablauf auf einem Mikroprozessor (20) die Verfahrensschritte nach einem der Ansprüche 1 bis 6 ausführt.

## Revendications

1. Procédé implémenté sur ordinateur pour commander un reflux dans un système microchirurgical (10), **caractérisé en ce qu'**il comprend les étapes consistant à :
fournir un signal afin d'effectuer une opération de pompe (14) pour fournir un liquide d'irrigation (13) à un débit constant vers un orifice occlus (32) d'un dispositif chirurgical (18) ;
utiliser un capteur de pression (16) pour détecter une pression de reflux à proximité dudit orifice (32) au fil du temps ; et
surveiller ladite pression de reflux en fonction du temps afin de détecter une rupture d'occlusion.

2. Procédé selon la revendication 1, dans lequel ladite étape de surveillance comprend la surveillance de l'inclinaison d'une courbe pression de reflux/temps (40).

3. Procédé selon la revendication 2, dans lequel ladite étape de surveillance de ladite inclinaison comprend la détection d'un changement de signe de ladite inclinaison.

4. Procédé selon la revendication 3, dans lequel ladite inclinaison passe d'un signe positif à un signe négatif.

5. Procédé selon la revendication 3, dans lequel ladite inclinaison passe d'un signe positif à zéro.

6. Procédé selon la revendication 1, dans lequel ladite étape de mise en marche commence lors de la détection d'une occlusion dudit orifice.

7. Programme informatique qui, lorsqu'il est exécuté sur un microprocesseur (20), est configuré pour réaliser les étapes de procédé selon l'une quelconque des revendications 1 à 6.
